# EUROPÄISCHE PATENTSCHRIFT

(11) **EP 1 338 249 B1**
(45) Veröffentlichungstag und Bekanntmachung des Hinweises auf die Patenterteilung: **02.06.2010**
(21) Anmeldenummer: 03003578.6
(22) Anmeldetag: 17.02.2003
(51) Int. Cl.: A61B 17/22

(54) **Medizinisches System**
Medical system
Système médical

(30) Priorität: 21.02.2002 DE 10207385
(43) Veröffentlichungstag der Anmeldung: 27.08.2003
(73) Patentinhaber: HealthTronics Inc., Marietta GA 30062 (US)
(72) Erfinder: Brill, Norbert, 78465 Konstanz (DE); van Rijn, Dick, 88145 Hergatz (DE); Senn, Wilfried, 8280 Kreuzlingen (CH); Bauer, Manfred, 8280 Kreuzlingen (CH); Freidinger, Jörg, 78351 Bodman-Ludwigshafen (DE)
(74) Vertreter: Mussgnug, Bernd

(56) Entgegenhaltungen:
- DE-A- 4 441 939
- DE-A- 19 520 748
- DE-A- 19 702 829
- DE-C- 4 232 683
- US-A- 5 044 354
- US-A- 6 036 661

## Beschreibung

Die Erfindung betrifft ein medizinisches System gemäß dem Oberbegriff des Anspruchs 1.

Ein medizinisches System dieser Gattung ist aus der DE 197 46 956 C2 bekannt. Dieses bekannte System dient zur Behandlung eines Patienten mit fokussierten akustischen Wellen. Der Patient wird auf einer Lagerungseinrichtung in Form eines Tisches gelagert. Der zu behandelnde Körperbereich des Patienten, z. B. ein Konkrement oder eine Gewebepartie wird mittels einer Ortungseinrichtung ermittelt. Eine Therapieeinrichtung erzeugt akustische Wellen, die auf den mittels der Ortungseinrichtung ermittelten Körperbereich fokussiert werden, um dort die gewünschte therapeutische Wirkung zu verursachen, z. B. ein Konkrement oder einen Tumor zu zerstören.

Bei dem bekannten System sind die Ortungseinrichtung und die Therapieeinrichtung jeweils gesonderte Geräte, die mit jeweils einer eigenen Versorgungseinrichtung ausgestattet sind. Die Ortungseinrichtung ist als Röntgengerät mit einem C-Bogen ausgebildet. Damit das System eingesetzt werden kann, werden die Ortungseinrichtung und die Therapieeinrichtung mechanisch gekoppelt und gegenseitig justiert, so das der Fokus der von der Therapieeinrichtung erzeugten akustischen Wellen in das Isozentrum der Röntgen-Ortungseinrichtung fällt.

Die Ortungseinrichtung und die Therapieeinrichtung sind relativ große und schwere Geräte. Die Kopplung und insbesondere die gegenseitige Justage und elektrische Verbindung der Ortungseinrichtung und der Therapieeinrichtung sind mühsam, zeitaufwendig und müssen durch Fachpersonal durchgeführt werden. Ein Transport des Systems, wozu die einzelnen Komponenten voneinander getrennt werden müssen, wird daher nur in Ausnahmefällen durchgeführt werden.

Als weiterer Stand der Technik werden die DE 195 20 748 A1, DE 42 32 683 C1, DE 197 02829 A1 und DE 44 41 939 A1 genannt.

Der Erfindung liegt die Aufgabe zugrunde, ein medizinisches System der eingangs genannten Gattung zu schaffen, welches in Bezug auf seine räumlichen Einsatzmöglichkeiten flexibler ist.

Diese Aufgabe wird erfindungsgemäß gelöst durch ein medizinisches System mit den Merkmalen des Anspruchs 1.

Vorteilhafte Ausführungen der Erfindung sind in den Unteransprüchen angegeben.

Der wesentliche Gedanke der Erfindung besteht darin, dass die Ortungseinrichtung, die Therapieeinrichtung mit ihren jeweils zugehörigen Versorgungseinrichtungen sowie die Lagerungseinrichtung in einem gemeinsamen Geräteträger angeordnet und montiert sind. Die Länge und die Breite des in den Geräteträger montierten Systems sind höchstens so groß wie die projizierte Grundfläche eines Standart-Krankenhausbettes, d.h. übertreffen diese Grundflächenabmessungen zumindest nicht wesentlich. Die Ortungseinrichtung, die Therapieeinrichtung und die Lagerungseinrichtung sind in dem Geräteträger fest montiert und relativ zueinander justiert. Die sich hieraus ergebende geringe Grundfläche ermöglicht es, das System in vollständig montiertem Zustand zu transportieren und insbesondere auch in die Behandlungsräume einer Klinik oder einer Arztpraxis in montiertem Zustand zu befördern. Dadurch ergibt sich der wesentliche Vorteil, dass das System werkseitig montiert und justiert angeliefert und aufgestellt werden kann, ohne dass am Aufstellungsort umfangreiche Montage- und Justierungsarbeiten erforderlich sind. Weiter kann das System in montiertem Zustand von einem Behandlungsort zu einem anderen Behandlungsort transportiert werden, ohne dass eine Entkopplung der einzelnen Geräte notwendig ist, die wiederum eine Kopplung und Justierung am neuen Aufstellungsort zur Folge hat. Das System kann dadurch in bequemer Weise von einem Raum in einen anderen Raum eines Gebäudes verbracht werden und eignet sich insbesondere auch für den mobilen Einsatz, bei welchem das System unterschiedlichen Nutzern an unterschiedlichen Orten jeweils für eine beschränkte Zeit z.B. angemietet zur Verfügung steht.

Die Lagerungseinrichtung des erfindungsgemässen medizinischen Systems ist zweckmäßigerweise verstellbar, so dass der auf der Lagerungseinrichtung liegende Patient mit seinem zu behandelnden Körperbereich in den Fokus der Therapieeinrichtung positioniert werden kann. Ist die Lagerungseinrichtung mit einem Liegestuhl ausgebildet, auf welchem der zu behandelnde Patient sitzt, so kann dieser Liegestuhl im Wesentlichen in die Außenabmessungen des Geräteträgers eingefügt werden, so dass die einfache Transportmöglichkeit des Systems durch die Lagerungseinrichtung nicht beeinträchtigt wird. In einer vorteilhaften Ausführung weist die Lagerungseinrichtung einen Liegetisch auf, auf welchem der Patient während der Behandlung ausgestreckt liegt. Damit die Transportmöglichkeiten des Systems nicht behindert werden, ist in dieser Ausführungsform der Liegetisch so ausgebildet, dass er vorzugsweise ohne Zuhilfenahme von Werkzeugen verkleinert werden kann. Hierzu ist die Liegefläche des Liegetisches zweckmäßigerweise unterteilt, wobei die einzelnen Flächenteile zusammengeschoben, zusammengeklappt oder hochgeschwenkt werden können, so dass sie nicht mehr über den Außenumfang des Geräteträgers hinausragen.
Vorzugsweise ist der Liegetisch der Lagerungseinrichtung an einer Schmalseite des Geräteträgers und quer zu dessen Längserstreckung angeordnet. Dadurch ist der Liegetisch von wenigstens drei Seiten frei zugänglich, so dass auch ein gebrechlicher oder behinderter Patient sich einfach auf den Liegetisch begeben kann bzw. von dem Pflegepersonal auf den Liegetisch gelegt werden kann. Außerdem ist der auf dem Liegetisch liegende Patient für den behandelnden Arzt bzw. das behandelnde Personal gut zugänglich. Die Anordnung der Lagerungseinrichtung an einer Endschmalseite des Geräteträgers macht außerdem möglich, dass der Liegetisch mit einem großen vertikalen Hubweg ausgestattet werden kann, wobei insbesondere ein Absenken der Liegefläche des Liegetisches zur Erleichterung des Besteigens des Liegetisches möglich ist. Hierzu kann der Liegetisch beispielsweise soweit gesenkt werden, dass die Liegefläche eine Aufstiegshöhe über dem Fußboden von weniger als 75 cm aufweist.

Um eine möglichst kompakte Anordnung des Systems in dem Geräteträger zu erreichen, sind die Versorgungseinrichtungen für die Therapieeinrichtung ,die Ortungseinrichtung und die Lagerungseinrichtung in einem Endbereich des Geräteträgers angeordnet und die Ortungseinrichtung und die Therapieeinrichtung sind an der schmalen Stirnseite der Versorgungseinrichtung angebracht, die der Lagerungseinrichtung zugewandt ist. Die Ortungseinrichtung ist i.d.R. schwenkbar gelagert, um den zu behandelnden Körperbereich räumlich zu lokalisieren und in dem Isozentrum der Ortungseinrichtung zu positionieren. Ist die Ortungseinrichtung an der schmalen Stirnseite der Versorgungseinrichtung angeordnet, so verläuft die Schwenkachse der Ortungseinrichtung vorzugsweise horizontal in Längsrichtung des Geräteträgers. Ist die Ortungseinrichtung insbesondere als Röntgeneinrichtung mit einer Röntgenstrahlquelle und einem Röntgenstrahlempfänger an einem schwenkbaren C-Bogen ausgebildet, so ist dieser C-Bogen vorzugsweise seitlich abgekröpft. Dadurch können die Schwenkachse des C-Bogens und die Stoßwellenquelle der Therapieeinrichtung in einer vertikalen Ebene übereinander liegen und der C-Bogen wird seitlich um die Stoßwellenquelle herumgeführt.

Die elektrische Energieversorgung und Steuerung der Ortungseinrichtung, der Therapieeinrichtung und der Lagerungseinrichtung sind in dem Geräteträger in einer kompakten Einheit integriert. Dies hat den Vorteil, dass die benötigten elektrischen Komponenten wesentlich reduziert werden können, dass eine gemeinsame Verkabelung installiert werden kann und eine elektrische Abstimmung von Ortungseinrichtung, Therapieeinrichtung und Lagerungseinrichtung und deren Versorgungseinrichtungen nicht notwendig ist. Weiter kann die gesamte Steuerung der Ortungseinrichtung, der Therapieeinrichtung und der Lagerungseinrichtung über ein Steuer- und Datenbussystem erfolgen. Das Steuer- und Datenbussystem kann über eine einzige Schnittstelle mit einer Bedieneinrichtung verbunden sein, über welche die Ortungseinrichtung, die Therapieeinrichtung und die Lagerungseinrichtung gesteuert und betätigt werden. Die Bedieneinrichtung kann als eine separate Einheit ausgebildet sein, welche leitungsgebunden oder nicht leitungsgebunden an das Steuer- und Datenbussystem angeschlossen ist. Die Bedieneinrichtung kann auch in das System integriert in dem Geräteträger angeordnet sein.

Die Integration der gesamten elektrischen und elektronischen Komponenten und insbesondere deren Installation in einem Steuer- und Datenbussystem ermöglichen weiter eine Ferndiagnose und/oder -wartung des gesamten Systems, z.B. über ein GSM-Modem oder ein Telefonmodem. Dadurch ist Wartung und Service des Systems zeit- und kostensparend möglich.

Die Ortungseinrichtung ist zweckmäßig mit einer bildgebenden Einrichtung ausgestattet, die vorzugsweise mit einem in die Bedieneinrichtung integrierten Monitor versehen ist, so dass der zu behandelnde Körperbereich des Patienten und dessen Positionierung von dem Arzt beobachtet und begutachtet werden können. Der Patient kann je nach dem zu behandelnden Körperbereich unterschiedlich auf der Lagerungseinrichtung gelagert werden. Insbesondere ist alternativ eine Rücken- oder Bauchlagerung möglich und ebenso ist eine Vertauschung von Kopfund Fußlage des Patienten z.B. zur Ortung und Behandlung der rechten oder linken Niere möglich. In einer vorteilhaften Ausführung ist die bildverarbeitende Einrichtung so ausgelegt, dass durch Eingabe der Lage des Patienten Spiegelungen der Bildinformationen in der Weise stattfinden, dass auf dem Monitor der Bedieneinrichtung stets die Abbildung in derselben Patientenlage erfolgt, z.B. in der Weise, dass der Körper des Patienten von vorn gesehen wird und der Kopf des Patienten sich im Bild oben befindet. Die die unterschiedlichen Positionen des Patienten auf der Lagerungseinrichtung kompensierenden Spiegelungen können außerdem auch auf die Steuerung der Lagerungseinrichtung durch die Bedieneinrichtung übertragen werden. Dadurch kann der Arzt an der Bedieneinrichtung die Positionierung nach dem ggf. gespiegelten Monitorbild durchführen, wobei die Lagerungseinrichtung entsprechend der tatsächlichen Position des Patienten gesteuert bewegt wird.

Das gesamte medizinische System kann mit dem Geräteträger einfach transportiert und stationär aufgestellt werden. Ist eine Veränderung des Aufstellungsortes beabsichtigt, so ist vorzugsweise der Geräteträger verfahrbar ausgebildet. Der Geräteträger weist hierzu an seinem einen Ende Laufrollen oder -räder auf. Mit dem anderen Ende sitzt der Geräteträger auf dem Fußboden auf, so dass das System stabil ortsfest steht. Für den Transport wird ein Fahrgerät verwendet, welches an dem den Laufrollen oder -rädern entgegengesetzten Ende des Geräteträgers angreift, um den Geräteträger mit dem integrierten System insgesamt verfahrbar zu machen. Das Fahrgerät untergreift dabei vorzugsweise formschlüssig den Geräteträger, um diesen vom Fußboden abzuheben. Das Fahrgerät ist steuerbar, um ein leichtes Manövrieren beim Verfahren zu ermöglichen. Zweckmäßigerweise kann das Fahrgerät auch motorisch angetrieben sein, z.B. mittels eines akkugespeisten Elektromotors.

Im Folgenden wird die Erfindung anhand von in der Zeichnung dargestellten Ausführungsbeispielen näher erläutert. Es zeigen
- Figur 1: eine Seitenansicht des medizinischen Systems in einer ersten Ausführung,
- Figur 2: eine Stirnansicht des Systems der Figur 1,
- Figur 3: eine Draufsicht auf das System in der zweiten Ausführung,
- Figur 4: eine Seitenansicht des Systems in der zweiten Ausführung während des Transports,
- Figur 5: eine Stirnansicht der Figur 4,
- Figur 6: eine Seitenansicht der Bedieneinrichtung,
- Figur 7: eine Stirnansicht der Bedieneinrichtung und
- Figur 8: eine Seitenansicht des Systems in einer dritten Ausführung mit integrierter Bedieneinrichtung.

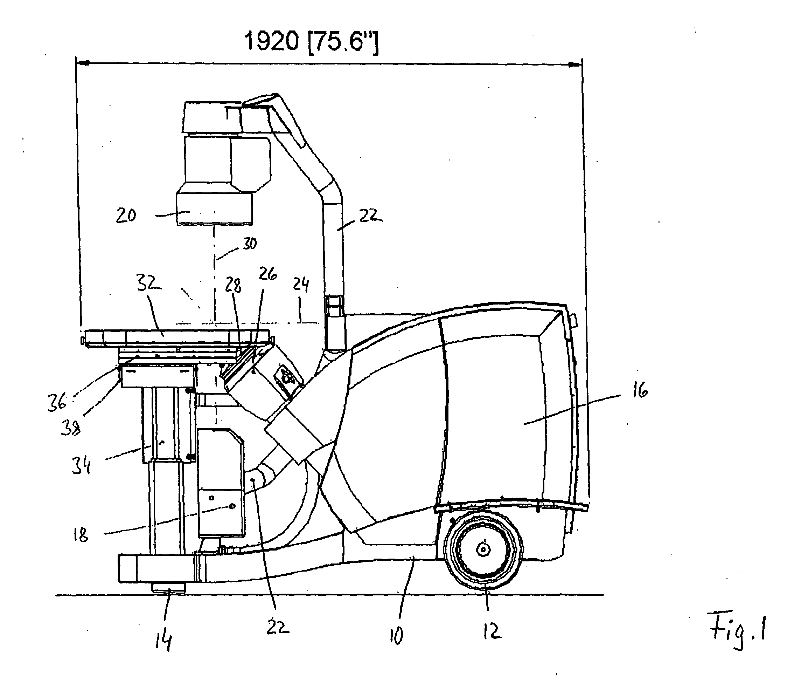

Figur 1 zeigt das medizinische System in einer ersten Ausführung.

Ein Geräteträger 10 trägt das gesamte System. Der Geräteträger 10 hat im Wesentlichen die Form eines horizontalen Rechtecks und wird durch eine einfache einteilige leichte Stahlbaustruktur gebildet. An seinem einen Schmalende weist der Geräteträger 10 Laufräder 12 auf. An dem anderen Schmalende steht der Geräteträger 10 mit Füßen 14 auf dem Fußboden auf.

Auf dem Geräteträger 10 sitzt ein Gehäuse 16, welches etwa die Hälfte der Längserstreckung des Geräteträgers einnimmt. An der Stirnschmalseite des Gehäuses 16 ist eine Einrichtung zur Ortung und/oder Diagnose (kurz Ortungseinrichtung) eines zur behandelnden Körperbereichs eines Patienten, eine Einrichtung zur Erzeugung und Fokussierung akustischer Wellen (Kurztherapieeinrichtung) auf diesen zu behandelnden Körperbereich und eine Einrichtung zur Lagerung des zu behandelnden Patienten (kurz Lagerungseinrichtung) angeordnet.

Die Ortungseinrichtung ist im dargestellten Ausführungsbeispiel eine Röntgen-Ortungseinrichtung, die aus einer Röntgenstrahlquelle 18 und einem Röntgenstrahlempfänger 20 besteht. Die Röntgenstrahlquelle 18 und der Röntgenstrahlempfänger 20 sind an den beiden freien Enden eines C-Bogens 22 angebracht. Der C-Bogen 22 ist in seinem mittleren Bereich um eine Schwenkachse 24 schwenkbar an dem Gehäuse 16 gelagert. Die Schwenkachse 24 verläuft horizontal und in Richtung der Längsachse des Geräteträgers 10. Die Funktionsweise einer solchen Röntgenortungseinrichtung ist an sich bekannter Stand der Technik.

Anstelle einer Röntgenortungseinrichtung, wie sie in der Zeichnung dargestellt ist, kann auch eine andere an sich bekannte Ortungseinrichtung verwendet werden, z.B. eine Ultraschall-Ortungseinrichtung.

Die Therapieeinrichtung weist einen Therapiekopf 26 auf. In dem Therapiekopf 26 werden z.B. durch eine elektrische Funkenentladung in einer Flüssigkeit akustische Druckwellen erzeugt, die z.B. durch einen Parabolreflektor als Stoßwellen fokussiert werden. Der Therapiekopf 26 ist durch eine Koppelmembran 28 abgeschlossen, die an den Körper des Patienten angelegt wird, um die in dem Therapiekopf 26 erzeugten akustischen Stoßwellen in den Körper des Patienten zu übertragen.

Die Achse 30 der von der Röntgenstrahlquelle 18 zu dem Röntgenstrahlempfänger 20 ausgesandten Röntgenstrahlen schneidet die Schwenkachse 24 des C-Bogens in einem Isozentrum. Dieses Isozentrum bleibt somit räumlich fest, wenn der C-Bogen 22 um die Schwenkachse 24 geschwenkt wird. Der Therapiekopf 26 ist an dem Gehäuse 16 fest angeordnet und so ausgerichtet, dass seine Mittelachse das Isozentrum schneidet und der Fokus der akustischen Stoßwellen in dieses Isozentrum fällt.

Die Lagerung des C-Bogens 22 mit der Schwenkachse 24 liegt in einer vertikalen Ebene, die in der Längsmittelachse des Geräteträgers 10 verläuft, oberhalb des Therapiekopfes 26. Deshalb ist der C-Bogen 22 zwischen der Schenkachse 24 und der Röntgenstrahlquelle 18 seitlich ausgekröpft und in einem Bogen um den Therapiekopf 26 herumgeführt.

Das Gehäuse 16 nimmt die elektrische Versorgung und die elektronische Steuerschaltung der Ortungseinrichtung und der Therapieeinrichtung auf. Ebenso kann in dem Gehäuse 16 ggf. eine Aufbereitungseinrichtung für das flüssige Medium des Therapiekopfes 26 angeordnet sein. Die elektronischen Schaltungen und Steuerungen der Versorgungseinrichtungen sind über ein gemeinsames Steuer und Datenbus-System zusammengefasst.

Die Lagerungseinrichtung weist einen horizontalen Liegetisch 32 auf, auf welchem der zu behandelnde Patient gelagert wird. Der Liegetisch 32 ist mittels teleskopisch ausfahrbarer vertikaler Hubsäulen 34 höhenverstellbar. Außerdem ist der Liegetisch 32 mittels horizontaler Führungen 36 gegen die Therapieeinrichtung hin und von dieser weg bewegbar. Weitere zu den Führungen 36 senkrechte horizontale Führungen 38 dienen dazu, den Liegetisch 32 quer zur Längserstreckung des Geräteträgers 10 zu verschieben. Die Höhenverstellung mittels der Hubsäulen 34 und die Horizontalverstellung in den Führungen 36 und 38 erfolgt vorzugsweise über einen pneumatischen, oder hydraulischen oder elektrischen Antrieb.

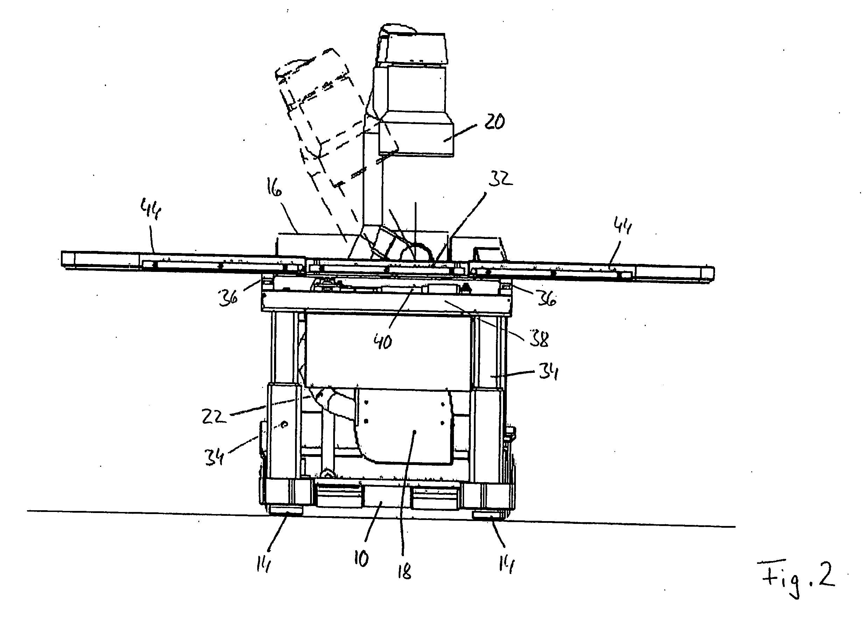

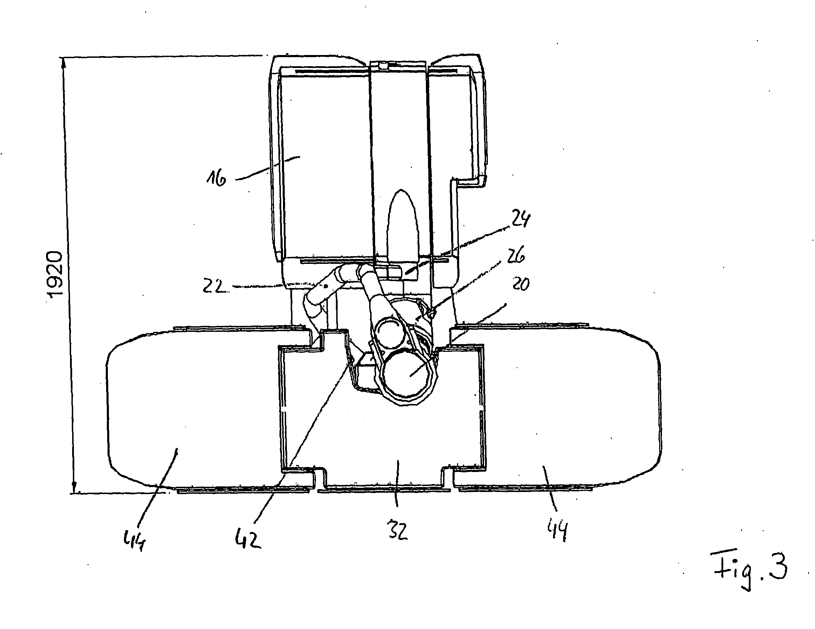

In Figur 2 ist ein pneumatisches Aggregat 40 für die horizontale Querverschiebung in den Führungen 38 erkennbar.
Der Liegetisch 32 weist in seinem mittleren Bereich an der dem Gehäuse 16 zugewandten Kante eine Aussparung 42 auf, wie in Figur 3 dargestellt. Der Patient wird auf dem Liegetisch 32 so gelagert, dass sein zu behandelnder Körperbereich z.B. ein zu zerstörendes Körperkonkrement, ein Tumor, ein zu behandelnder Knochenbereich oder dergleichen auf dieser Aussparung 42 zu liegen kommt. Mittels der Hubsäulen 34 und der Führungen 36 und 38 kann der Liegetisch 32 mit dem Patienten in den drei räumlichen Koordinaten gesteuert verfahren werden, um den mittels der Ortungseinrichtung lokalisierten zu behandelnden Körperbereich in dem Isozentrum und damit in dem Fokus des Therapiekopfes 26 zu positionieren. Hierzu werden die Signale der Ortungseinrichtung, z.B. die Signale des Röntgenstrahlempfängers 20 zu einer Bildverarbeitung geleitet und in einem bildgebenden System, z.B. auf einem Monitor dargestellt. Durch Schwenken des C-Bogens 22 ist eine Beobachtung unter unterschiedlichen räumlichen Richtungen möglich, um die räumliche Lage des zu behandelnden Körperbereichs zu ermitteln.

In Figur 2 ist auch gestrichelt dargestellt, wie die Röntgenortungseinrichtung aus der vertikalen Position um 30° geschwenkt wird, um den zu behandelnden Körperbereich aus zwei räumlichen Richtungen anzupeilen und zu lokalisieren.

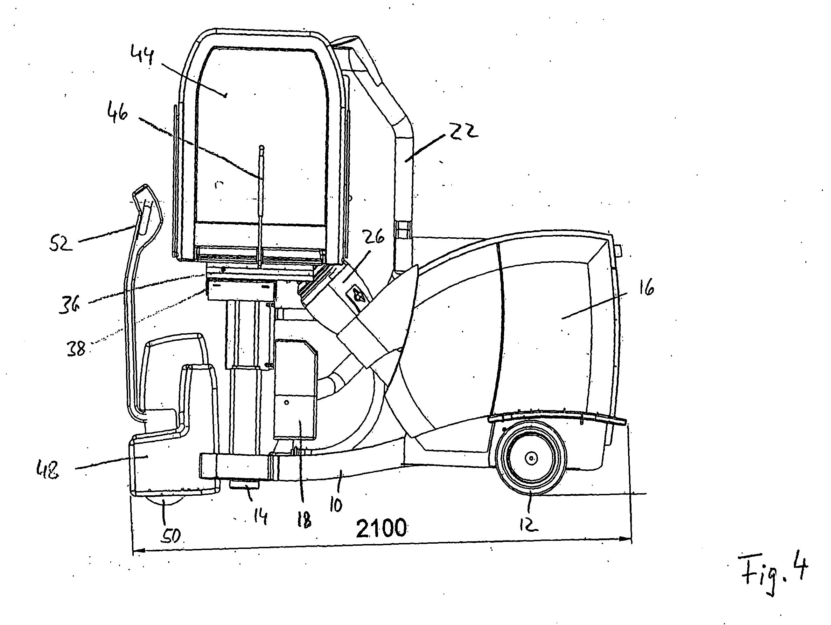

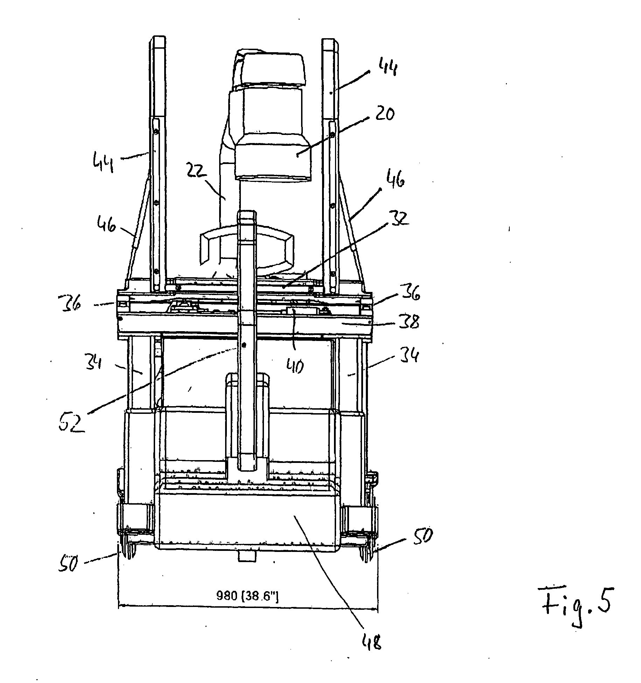

In den Figuren 2 und 3 ist die Liegefläche des Liegetisches 32 in drei Flächenteile unterteilt dargestellt. Der mittlere Flächenteil mit der Aussparung 42 ist für die Positionierung des Patienten in den Führungen 36 und 38 verschiebbar gelagert. Die beiden über den Geräteträger 10 beidseitig hinausragenden Endteile 44 der Liegefläche sind jeweils um eine horizontale in Längsrichtung des Geräteträgers 10 verlaufende Achse hochschwenkbar, bis sie senkrecht nach oben stehen und sich somit innerhalb der Grundfläche des Geräteträgers 10 befinden. Zum Hochschwenken der Endteile 44 dienen ebenfalls pneumatische Aggregate 46. Während in den Figuren 1, 2 und 3 die Endteile 44 nach unten geklappt sind, so dass der Liegetisch 32 eine durchgehende horizontale Liegefläche aufweist, zeigen die Figuren 4 und 5 die Endteile 44 in der hochgeschwenkten Stellung.

Die Länge und die Breite des Gerätesystems entsprechen der Grundfläche eines Standard-Krankenhausbettes oder sind ggf. auch kleiner als diese Grundfläche. Dementsprechend ist in den Figuren 1 und 3 die Gesamtlänge des auf dem Geräteträger 10 angeordneten integrierten Systems mit 1920 mm angegeben und in Figur 5 die Breite des Systems mit den hochgeschwenkten Endteilen 44 mit 980 mm.

In der in der Zeichnung dargestellten Ausführung, in welcher der Geräteträger 10 mit Laufrädern 12 versehen ist, ist das System fahrbar. Wie in den Figuren 4 und 5 dargestellt ist, kann hierzu ein Fahrgerät 48 verwendet werden, welches an der den Laufrädern 12 entgegengesetzten Schmalseite des Geräteträgers 10 angesetzt wird und den Geräteträger 10 untergreift, um den Geräteträger 10 anzuheben, so dass die Füße 14 vom Fußboden freikommen. Vorzugsweise greift das Fahrgerät 48 dabei formschlüssig an der Unterseite des Geräteträgers 10 an. Das Fahrgerät 48 ist mit Laufrädern 50 ausgestattet und vorzugsweise motorisch angetrieben, z.B. durch einen akkugespeisten Elektromotor. Ein Handgriff 52 dient zum Führen und Manövrieren des auf den Laufrädern 12 des Geräteträgers 10 und den Laufrädern 15 des Fahrgeräts 48 verfahrbaren Systems. Aufgrund der oben genannten Abmessungen des Geräteträgers 10 und des auf dem Geräteträger angeordneten integrierten System ist ein Verfahren und Manövrieren des Systems auch im Inneren von Gebäuden und durch Türöffnungen möglich.

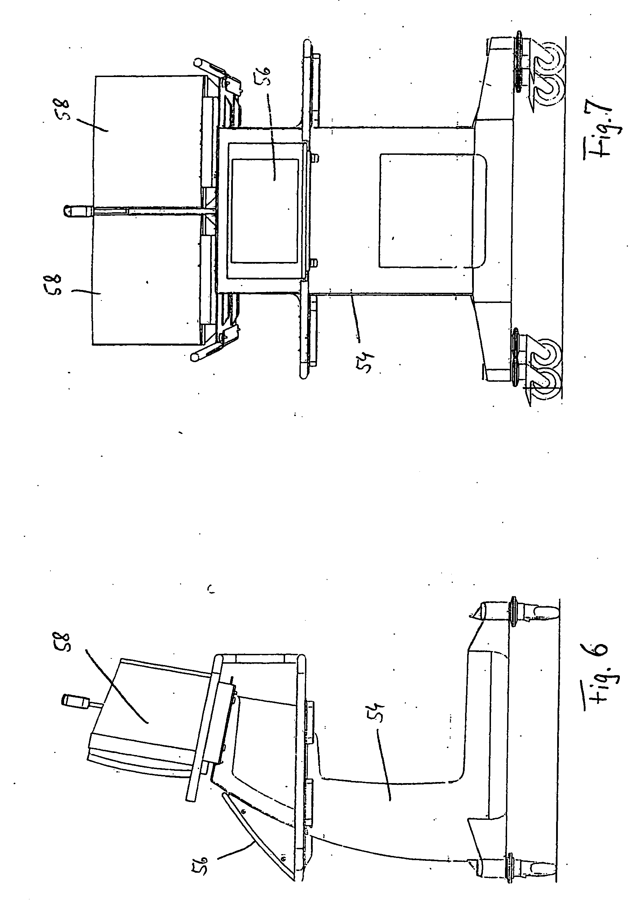

In den Ausführungen der Figuren 1 bis 5 wird das System mit einer separaten Bedieneinrichtung 54 betrieben, die in den Figuren 6 und 7 dargestellt ist. Die Bedieneinrichtung 54 ist als selbstständige verfahrbare Einheit ausgebildet. Die Bedieneinrichtung 54 weist ein Bedienfeld 56 auf, das bsw. als Touch-Screen realisiert sein kann. Das Bedienfeld 56 enthält Schaltelemente für den Betrieb und die Steuerung der Ortungseinrichtung und der Therapieeinrichtung sowie Schaltelemente für das Verfahren des Liegetisches 32 in den drei räumlichen Achsen. Weiter weist die Bedienungseinrichtung 54 Anzeigeeinrichtungen, z.B. Monitore 58 auf, um die von der Ortungseinrichtung aufgenommenen Bilder darzustellen. Die Bedieneinrichtung 54 ist mit dem System über Kabel oder über einen nicht leitungsgebundenen Signalaustausch verbunden.

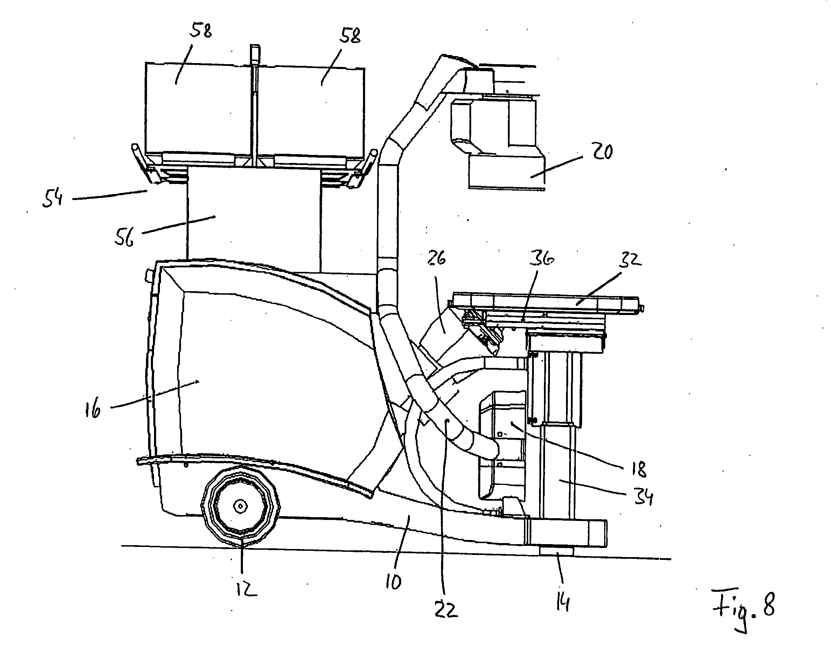

Figur 8 zeigt eine weitere Abwandlung des Systems, bei welcher auch die Bedieneinrichtung 54 auf dem Geräteträger 10 angeordnet und in das System integriert ist. Hierzu ist die Bedieneinrichtung 54 auf dem Gehäuse 16 aufgebaut, wodurch sich eine weitere Platzeinsparung und eine Erleichterung des Transports ergibt.

Bezugszeichenliste
- 10: Geräteträger
- 12: Laufräder
- 14: Füße
- 16: Gehäuse
- 18: Röntgenstrahlquelle

- 20: Röntgenstrahlempfänger
- 22: C-Bogen
- 24: Schwenkachse
- 26: Therapiekopf
- 28: Koppelmembran

- 30: Achse für Röntgenstrahlen
- 32: Liegetisch
- 34: Hubsäulen
- 36: Führungen
- 38: Führungen

- 40: Pneumatisches Aggregat
- 42: Aussparung
- 44: Endteile
- 46: Pneumatische Aggregate
- 48: Fahrgerät

- 50: Laufräder
- 52: Handgriff
- 54: Bedieneinrichtung
- 56: Bedienfeld
- 58: Monitore

## Patentansprüche

1. Medizinisches System, mit einer Ortungseinrichtung zur Ortung und/oder Diagnose eines zu behandelnden Körperbereichs eines Patienten, mit einer Therapieeinrichtung zur Erzeugung von akustischen Wellen und zur Fokussierung dieser Wellen in den zu behandelnden Körperbereich, mit einer Lagerungseinrichtung zur Lagerung des zu behandelnden Patienten und mit Versorgungseinrichtungen zur Energieversorgung und Steuerung der Ortungseinrichtung, der Therapieeinrichtung und der Lagerungseinrichtung, wobei die Ortungseinrichtung (18, 20, 22), die Therapieeinrichtung (26), die Lagerungseinrichtung (32), und deren Versorgungseinrichtungen in einem gemeinsamen Geräteträger (10) angeordnet sind und wobei die Ortungseinrichtung (18, 20) und die Therapieeinrichtung (26) in diesem Geräteträger (10) relativ zueinander justiert sind, **dadurch gekennzeichnet, dass** die projizierte Grundfläche der Ortungseinrichtung (18, 20, 22), der Therapieeinrichtung (26), der Lagerungseinrichtung (32), und deren Versorgungseinrichtungen eine Länge von 1920 mm und eine Breite von 980 mm aufweist, wobei der Geräteträger (10) an einem Ende Laufräder (12) aufweist und an seinem entgegengesetzten Ende mit Füßen (14) auf dem Fußboden aufsteht und dass ein Fahrgerät (48) vorgesehen ist, mit welchem dieses andere Ende des Geräteträgers (10) angehoben werden kann, um den Geräteträger verfahrbar zu machen.

2. Medizinisches System nach Anspruch 1,
**dadurch gekennzeichnet, dass** die Lagerungseinrichtung (32), in Bezug auf die Ortungseinrichtung (18, 20) und die Therapieeinrichtung (26) räumlich positionierbar ist.

3. Medizinisches System nach den Ansprüchen 1 und 2,
**dadurch gekennzeichnet, dass** die Lagerungseinrichtung einen Liegetisch (32) aufweist, der in einer Betriebsstellung zur Lagerung eines Patienten über den Geräteträger (10) hinausragt, und der in eine Transportstellung gebracht werden kann, in welcher er sich innerhalb des Umfangs des Geräteträgers (10) befindet.

4. Medizinisches System nach einem der vorhergehenden Ansprüche,
**dadurch gekennzeichnet, dass** die Versorgungseinrichtungen der Ortungseinrichtung, der Therapieeinrichtung und der Lagerungseinrichtung in einem gemeinsamen Gehäuse (16) integriert und gemeinsam elektrisch verschaltet sind.

5. Medizinisches System nach Anspruch 4,
**dadurch gekennzeichnet, dass** die Steuerung und Überwachung der Ortungseinrichtung, der Therapieeinrichtung und der Lagerungseinrichtung über ein Steuer- und Datenbussystem erfolgt.

6. Medizinisches System nach Anspruch 4,
**dadurch gekennzeichnet, dass** die Ortungseinrichtung, die Therapieeinrichtung und die Lagerungseinrichtung über eine gemeinsame Bedieneinrichtung (54) überwacht und gesteuert werden.

7. Medizinisches System nach Anspruch 4,
**dadurch gekennzeichnet, dass** die Bedieneinrichtung (54) eine bildgebende Einrichtung (58) aufweist zur Darstellung der von der Ortungseinrichtung empfangenen Bildsignale.

8. Medizinisches System nach Anspruch 7,
**dadurch gekennzeichnet, dass** die Bildsignale der Ortungseinrichtung zu der bildgebenden Einrichtung (58) über eine Bildverarbeitung geführt werden, die entsprechend einer eingebbaren Patientenposition umschaltbar eine oder mehrere Bildspiegelungen durchführt.

9. Medizinisches System nach Anspruch 5,
**dadurch gekennzeichnet, dass** das Steuer und Datenbussystem an eine Ferndiagnose und/oder - Wartung anschließbar ist.

10. Medizinisches System nach einem der vorhergehenden Ansprüche,
**dadurch gekennzeichnet, dass** die Versorgungseinrichtungen in einem Gehäuse (16) integriert sind, dass die Ortungseinrichtung (18, 20) und die Therapieeinrichtung (26) an einer Stirnseite des Gehäuses (16) angeordnet sind und dass die Lagerungseinrichtung (32) vor dieser Stirnseite angeordnet ist.

11. Medizinisches System nach Anspruch 10,
**dadurch gekennzeichnet, dass** die Ortungseinrichtung eine Röntgenortungseinrichtung mit einer Röntgenstrahlquelle (18) und einem Röntgenstrahlempfänger (20) ist, die an einem C-Bogen (22) angeordnet sind, dass der C-Bogen in einer vertikalen Längsmittelebene oberhalb des Therapiekopfes (26) der Therapieeinrichtung schwenkbar gelagert ist und dass der C-Bogen (22) unterhalb seiner Schwenklagerachse bogenförmig ausgekröpft um den Therapiekopf (26) herumgeführt ist.

12. Medizinisches System nach Anspruch 11,
**dadurch gekennzeichnet, dass** der C-Bogen (22) der Ortungseinrichtung in der Höhe reduzierbar ausgeführt ist.

13. Medizinisches System nach Anspruch 12,
**dadurch gekennzeichnet, dass** der C-Bogen (22) der Ortungseinrichtung vorzugsweise klappbar ausgeführt ist.

## Claims

1. Medical system, comprising a locating device for locating and/or diagnosing a body region to be treated on a patient, comprising a therapy device for generating acoustic waves and for focusing these waves into the body region to be treated, comprising a support device for supporting the patient to be treated, and comprising supply devices for supplying power to and controlling the locating device, the therapy device and the support device, wherein the locating device (18, 20, 22), the therapy device (26), the support device (32) and the supply devices thereof are arranged in a common equipment carrier (10), and wherein the locating device (18, 20) and the therapy device (26) are adjusted relative to one another in this equipment carrier (10), **characterised in that** the projected base surface area of the locating device (18, 20, 22), of the therapy device (26), of the support device (32) and of the supply devices thereof has a length of 1920 mm and a width of 980 mm, wherein the equipment carrier (10) has wheels (12) at one end and rests on the floor via feet (14) at the opposite end, and **in that** a travel device (48) is provided, by means of which this other end of the equipment carrier (10) can be raised in order to make the equipment carrier displaceable.

2. Medical system according to claim 1, **characterised in that** the support device (32) can be spatially positioned in relation to the locating device (18, 20) and the therapy device (26).

3. Medical system according to claims 1 and 2, **characterised in that** the support device comprises a patient table (32) which protrudes beyond the equipment carrier (10) in an operating position for supporting a patient and which can be brought into a transport position in which it is located within the perimeter of the equipment carrier (10).

4. Medical system according to one of the preceding claims, **characterised in that** the supply devices of the locating device, of the therapy device and of the support device are integrated in a common housing (16) and are electrically interconnected.

5. Medical system according to claim 4, **characterised in that** the control and monitoring of the locating device, of the therapy device and of the support device takes place via a control and data bus system.

6. Medical system according to claim 4, **characterised in that** the locating device, the therapy device and the support device are monitored and controlled via a common control device (54).

7. Medical system according to claim 4, **characterised in that** the control device (54) has an imaging device (58) for displaying the image signals received from the locating device.

8. Medical system according to claim 7, **characterised in that** the image signals from the locating device are fed to the imaging device (58) via an image processing which carries out one or more image reflections in a manner that can be switched according to a patient position that can be input.

9. Medical system according to claim 5, **characterised in that** the control and data bus system can be connected to a remote diagnosis and/or maintenance system.

10. Medical system according to one of the preceding claims, **characterised in that** the supply devices are integrated in a housing (16), **in that** the locating device (18, 20) and the therapy device (26) are arranged at one end side of the housing (16), and **in that** the support device (32) is arranged in front of this end side.

11. Medical system according to claim 10, **characterised in that** the locating device is an X-ray locating device with an X-ray source (18) and an X-ray receiver (20) which are arranged on a C-arm (22), **in that** the C-arm is mounted such as to be able to pivot in a vertical longitudinal mid-plane above the therapy head (26) of the therapy device, and **in that** the C-arm (22) is guided in an arc-shaped curve around the therapy head (26) below its pivot bearing axis.

12. Medical system according to claim 11, **characterised in that** the C-arm (22) of the locating device is designed such as to be able to be reduced in height.

13. Medical system according to claim 12, **characterised in that** the C-arm (22) of the locating device is designed such that it can preferably be folded.

## Revendications

1. Système médical avec une installation de localisation pour localiser et/ou diagnostiquer une zone corporelle à traiter d'un patient avec une installation thérapeutique pour générer des ondes acoustiques et pour les focaliser dans la zone corporelle à traiter, comprenant :
une installation de positionnement pour positionner le patient à traiter, et
une installation d'alimentation pour l'alimentation en énergie et la commande de l'installation de localisation, de l'installation thérapeutique et de l'installation de positionnement,
système dans lequel
l'installation de localisation (18, 20, 22), l'installation thérapeutique (26), l'installation de positionnement (32) et leurs installations d'alimentation, sont logés dans un même support d'appareil (10) et l'installation de localisation (18, 20) et l'installation thérapeutique (26) sont ajustées l'une par rapport à l'autre dans ce support d'appareil (10),
**caractérisé en ce que**
la surface de base projetée de l'installation de localisation (18, 20, 22), de l'installation thérapeutique (26), de l'installation de positionnement (32) et de leurs installations d'alimentation, a une longueur de 1920 mm et une largeur de 980 mm,
le support d'appareil (10) a des roues (12) à l'une de ses extrémités et repose à son extrémité opposée par des pieds (14) sur le sol, et comporte
un appareil de déplacement (48) pour soulever l'autre extrémité du support d'appareil (10) et le rendre roulant.

2. Système médical selon la revendication 1,
**caractérisé en ce que**
l'installation de positionnement (32) peut être positionnée dans l'espace par rapport à l'installation de localisation (18, 20) et à l'installation thérapeutique (26).

3. Système médical selon la revendication 1 ou 2,
**caractérisé en ce que**
l'installation de positionnement à une table (32) qui, dans une position d'utilisation, vient en saillie pour positionner un patient au-dessus du support d'appareil (10) et qui peut être mise dans une position de transport dans laquelle elle se trouve à l'intérieur du périmètre du support d'appareil (10).

4. Système médical selon l'une des revendications précédentes,
**caractérisé en ce que**
l'installation d'alimentation de l'installation de localisation, l'installation thérapeutique et l'installation de positionnement, sont intégrés dans un même boîtier (16) et sont reliés électriquement.

5. Système médical selon la revendication 4,
**caractérisé en ce que**
la commande et la surveillance de l'installation de localisation, de l'installation thérapeutique et de l'installation de positionnement, se font par un système de bus de commande et de transmission de données.

6. Système médical selon la revendication 4,
**caractérisé en ce que**
l'installation de localisation, l'installation thérapeutique et l'installation de positionnement, sont surveillés et commandés par une même installation de commande (54).

7. Système médical selon la revendication 4,
**caractérisé en ce que**
l'installation de commande (54) présente une installation d'affichage (58) pour représenter les signaux vidéo reçus de l'installation de localisation.

8. Système médical selon la revendication 7,
**caractérisé en ce que**
les signaux vidéo de l'installation de localisation sont transmis à l'installation d'affichage (58) par une installation de traitement d'image qui, en fonction d'une position saisie du patient, peut commuter pour effectuer un ou plusieurs renversements d'images.

9. Système médical selon la revendication 5,
**caractérisé en ce que**
le système de bus de commande et de transmission de données est relié à une unité de diagnostic à distance et/ou d'entretien à distance.

10. Système médical selon l'une des revendications précédentes,
**caractérisé en ce que**
des installations d'alimentation sont intégrées dans le boîtier (16),
l'installation de localisation (18, 20) et l'installation thérapeutique (26) sont disposées à l'une des faces frontales du boîtier (16), et
l'installation de positionnement (32) est disposée devant cette face frontale.

11. Système médical selon la revendication 10,
**caractérisé en ce que**
l'installation de localisation est une installation de localisation pour radiographie comportant une source de rayons X (18) et un récepteur de rayons X (20) qui sont associés à un col de cygne (22), et
le col de cygne est pivotant dans un plan médian longitudinal vertical au-dessus de la tête de traitement (26) de l'installation thérapeutique, et
le col de cygne (22) est évidé sous son axe de basculement suivant une forme courbe pour contourner la tête (26) de l'installation thérapeutique.

12. Système médical selon la revendication 11,
**caractérisé en ce que**
le col de cygne (22) de l'installation de localisation, est réalisé avec une hauteur réductible.

13. Système médical selon la revendication 12,
**caractérisé en ce que**
le col de cygne (22) de l'installation de localisation est réalisé de préférence rabattable.
